# EUROPEAN PATENT APPLICATION

(11) **EP 2 626 095 A1**
(43) Date of publication of application: **14.08.2013**
(21) Application number: 12154562.8
(22) Date of filing: 08.02.2012
(51) Int. Cl.: A61M 5/24

(54) **Medicament delivery device**

(71) Applicant: Sanofi-Aventis Deutschland GmbH, 65929 Frankfurt am Main (DE)
(72) Inventor: The designation of the inventor has not yet been filed

(57) **Abstract**

Described is a medicament delivery device (5) comprising a housing including a proximal part (10) rotatably coupled to a distal part (15), a drive mechanism (60) disposed with the housing and adapted to apply force on a plunger (65) for dispensing a medicament, and a reset mechanism disposed within the housing. Rotation of the proximal part (10) relative to the distal part (15) from a first angular position to a second angular position activates the reset mechanism to reset the drive mechanism (60).

## Description

### Background of the Invention

Patients suffering from diseases like diabetes and rheumatoid arthritis and patients restricted to bed-rest or in post-operative settings typically have to receive medicaments via injection (whether self-administered or from another individual, e.g., a physician). Medicament delivery devices, such as pen injectors, auto-injectors, pumps and syringes, have been developed to facilitate injections. The medicament delivery devices may be disposable single-dose or multidose devices or may be reusable and utilize replacement medicament cartridges or syringes.

In some conventional medicament delivery devices, manual force is utilized to deliver the medicament. For example, a patient is required to push a plunger of the syringe to deliver the medicament. For patients with limited dexterity or limited movement, such a task may be difficult. Additionally, in such cases, patients may have difficultly setting a dose or ensuring that an entire dose has been delivered. Thus, there is a need for an improved medicament delivery device

### Summary of the Invention

It is an object of the present invention to provide an improved medicament delivery device.

In an exemplary embodiment, a medicament delivery device according to the present invention comprising a housing including a proximal part rotatably coupled to a distal part, a drive mechanism disposed with the housing and adapted to apply force on a plunger for dispensing a medicament, and a reset mechanism disposed within the housing. Rotation of the proximal part relative to the distal part from a first angular position to a second angular position activates the reset mechanism to reset the drive mechanism.

In an exemplary embodiment, the proximal part is coupled to the distal part via a hinge.

In an exemplary embodiment, the proximal part and the distal part are axially aligned in the first angular position and the second angular position.

In an exemplary embodiment, the proximal part is disposed at an angle to the distal part in the first angular position and the proximal part is axially aligned with the distal part in the second angular position.

In an exemplary embodiment, the housing is adapted to receive a cartridge or a syringe.

In an exemplary embodiment, the medicament delivery device further comprises a trigger button disposed on the housing. Axial movement of the trigger button relative to the housing activates the drive mechanism. Rotation of the trigger button relative to the housing affects the force applied by the drive mechanism.

In an exemplary embodiment, the reset mechanism includes a resilient, elastic beam biasing the proximal part and the distal part to the second angular position. The beam is grounded in the distal part and includes an operative end adapted to engage the drive mechanism. The operative end engages the drive mechanism to increase the force applied by the drive mechanism when the proximal part is rotated relative to the distal part from the first angular position to the second angular position. The beam prevents movement of at least one of the drive mechanism, the trigger button and the plunger when the proximal part is rotated relative to the distal part from the second angular position to the first angular position.

In an exemplary embodiment, the medicament delivery device further comprises a use indicator displaying a first indicia when the device is ready to be use and a second indicia when the device is locked. Rotation of the proximal part relative to the distal part from the first angular position to the second angular position causes the beam to engage the use indicator to display the first indicia.

In an exemplary embodiment, the medicament is one of insulin, complex carbohydrates, pharmaceutically active peptides, pharmaceutically active carbohydrates, pharmaceutically active nucleic acids, antibodies, a vaccine and a biological material.

In an exemplary embodiment, the drive mechanism is one or more of a spring, a pneumatic device a hydraulic device, a magnetic drive and a motor.

Further scope of applicability of the present invention will become apparent from the detailed description given hereinafter. However, it should be understood that the detailed description and specific examples, while indicating preferred embodiments of the invention, are given by way of illustration, since various changes and modifications within the spirit and scope of the invention will become apparent to those skilled in the art from this detailed description.

### Brief Description of the Drawings

- Figure 1: shows an exemplary embodiment of an medicament delivery device in a first angular position according to the present invention; and
- Figure 2: shows an exemplary embodiment of a medicament delivery device in a second angular position according to the present invention.

Corresponding parts are marked with the same reference symbols in all figures.

### Detailed Description

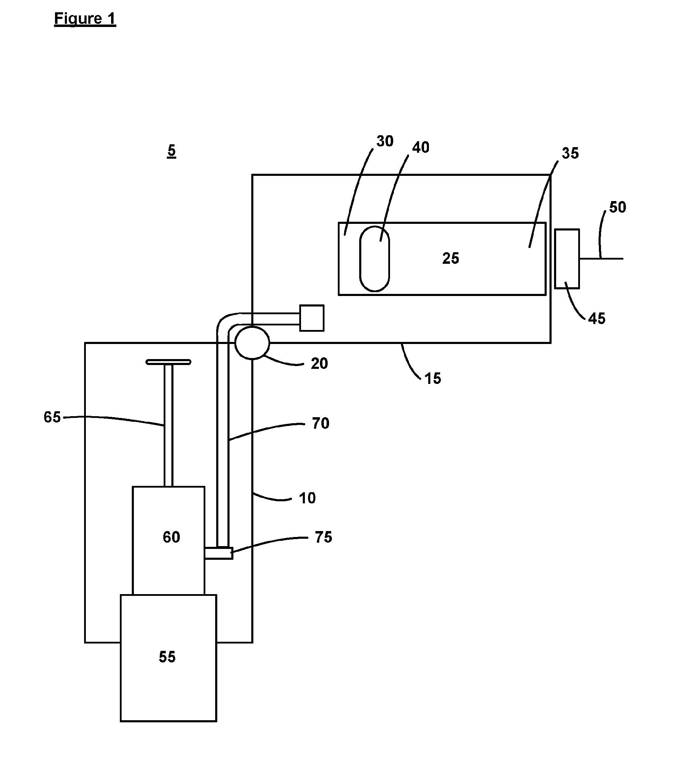

Figure 1 shows an exemplary embodiment of a medicament delivery device 5 according to the present invention. In the exemplary embodiment shown in Figure 1, the device 5 is depicted as a pen injector or an auto-injector. However, those of skill in the art will understand that the device 5 according to the present invention may include pen injectors, auto-injectors, pumps, syringes and other transdermal fluid delivery systems. For example, the device 5 may be utilized to deliver medicaments or other therapeutic agents into an intravenous port or a perfusion system.

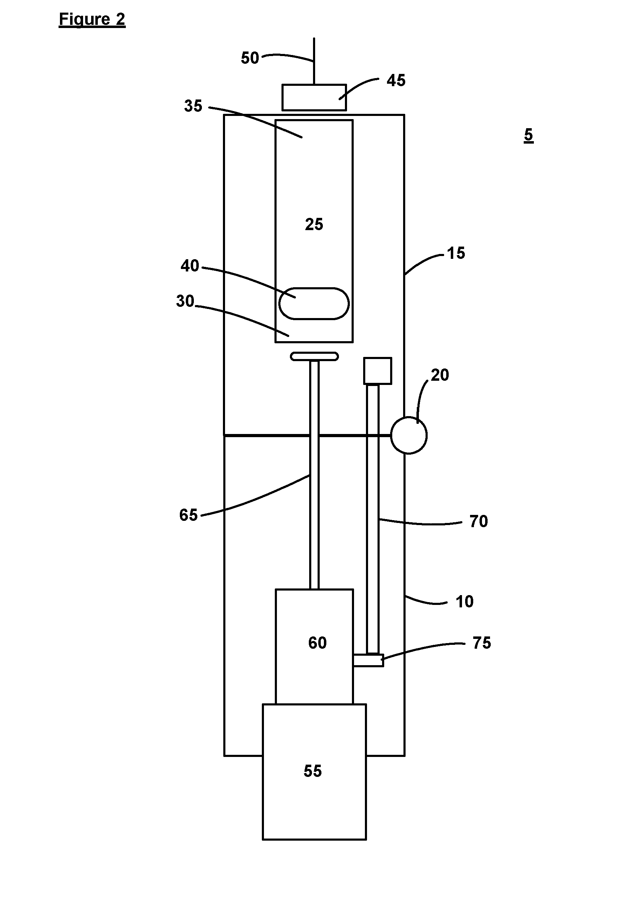

In the exemplary embodiment shown in Figure 1, the device 5 comprises a housing having a proximal part 10 and a distal part 15. The proximal and distal parts 10, 15 are coupled via a hinge 20. Thus, the proximal and distal parts, 10, 15 may rotate relative to each other via the hinge 20. In another exemplary embodiment, the proximal and distal parts 10, 15 may be axially aligned and rotate relative to each about a longitudinal axis A. Figure 1 shows a first angular position, in which the proximal part 10 is disposed at an angle (e.g., 45°) to the distal part 15. Figure 2 shows a second angular position in which the proximal part 10 is aligned with the distal part 15, forming an elongate housing, in which longitudinal axes of the parts 10, 15 lie substantially on a same longitudinal axis A. In the second angular position, the housing may have an elongate shape to facilitate usability, e.g., for a patient with limited dexterity. Thus, the housing may be elongate and ergonomically shaped (e.g., like a bicycle handlebar) to assist with gripping and holding the device 5.

Disposed within the distal part 15 of the housing may be a cartridge 25 containing a medicament. In an exemplary embodiment, the cartridge 25 includes a proximal portion 30 and a distal portion 35. A bung 40 may be slidably disposed in the cartridge 15 and initially disposed in the proximal portion 30 of the cartridge 25. As the bung 40 moves from the proximal portion 30 to the distal portion 35, the medicament in the cartridge 25 is dispensed.

In an exemplary embodiment, a needle assembly 45 may be removably coupled to the distal part 15 of the housing. The needle assembly 45 may include a needle hub coupled to a double-pointed needle 50 having a proximal tip adapted to penetrate a septum on the cartridge 25 and a distal tip adapted to pierce an injection site. Those of skill in the art will understand that in other exemplary embodiments, the cartridge 25 may be a pre-filled syringe with an integral needle or the device 5 may utilize a needleless injection arrangement (e.g., a nozzle) for delivering the medicament.

In an exemplary embodiment, the proximal part 10 of the housing may include a trigger button 55. Although the trigger button 55 is shown in the exemplary embodiment as disposed on a proximal end of the proximal part 10 of the housing, those of skill in the art will understand that the trigger button 55 may be disposed on a side surface of the proximal part 10 or the distal part 15 of the housing.

Actuation of the trigger button 55 releases a drive mechanism 60 disposed in the proximal part 10 of the housing. In an exemplary embodiment, the drive mechanism 60 is a spring, but those of skill in the art will understand that other drive mechanisms may be utilized, including but not limited to, tension springs, torsion springs, pneumatic devices, hydraulic devices, motors, magnetic drives, etc.

The drive mechanism 60 is operably coupled to a plunger 65. The plunger 65 is axially movable relative to the housing, and when force is applied by the drive mechanism 60 on the plunger 65, the plunger 65 engages the bung 40 in the cartridge 25 to dispense the medicament.

In an exemplary embodiment, the device 5 includes a dosing mechanism. In one exemplary embodiment, the dosing mechanism may include the trigger button 55 which is rotatably and slidably mounted on the proximal part 10 of the housing. The trigger button 55 may be used to "dial" a dose by, for example, rotating the trigger button 55 relative to the housing. As the trigger button 55 rotates in a first rotational direction, a potential dose may increase, and as the trigger button 55 rotates in a second rotational direction, a potential dose may decrease. Thus, if a dose is set too high or too low, it can be corrected prior to delivery. Rotation of the trigger button 55 may influence the force applied by the drive mechanism 60. For example, as the dose increases, the force applied by the drive mechanism 60 may increase to allow the plunger 65 to move the bung 40 a corresponding axial distance relative to the cartridge 25 to dispense the desired dose. In another exemplary embodiment, the dosing mechanism may be electronic or electro-mechanical, including, for example, an input device (e.g., a switch, a button, a dial) which can be manipulated to enter a dose on a display on the housing. When the dose has been entered, the drive mechanism 60 may perform a corresponding action to determine the force necessary to apply to the plunger 65 to displace the bung 40 to deliver the dose.

In another exemplary embodiment, the device 5 includes a reset mechanism. In an exemplary embodiment, the resent mechanism includes an elastic beam 70, grounded distally in the distal part 15 of the housing and having an operative end 75 disposed in the proximal part 10 of the housing. In an exemplary embodiment, the beam 70 may be limited to axial movement relative to the housing by being at least partially keyed to the housing or by abutting or being disposed between one or more rails formed on the housing. When the device 5 is in the first angular position as shown in Figure 1, the beam 70 may deflect around the hinge 20. In an exemplary embodiment, the beam 70 may be resilient and be biased in an axial orientation. Thus, when the device 5 is in the first angular position, the biasing force of the beam 70 attempts to return the device 5 to the second angular position shown in Figure 2.

In an exemplary embodiment, the beam 70 may be used, at least in part, to set the drive mechanism 60 for delivery of the desired dose. When the device 5 is moved from the first angular position to the second angular position, the operative end 75 of the beam 70 may engage the drive mechanism 60. For example, the operative end 75 of the beam 70 may engage the spring, compressing it a predetermined distance. The predetermined distance and a spring constant may be utilized to determine a force added to the spring when the device 5 moves from the first angular position to the second angular position. This additional force provided by the beam 70 may be taken into account when setting a dose and determining how much force is needed to advance the plunger 75 to deliver the desired dose. In an exemplary embodiment, the device 5 may be required to move from the first angular position to the second angular position prior to delivery of a dose. For example, after administration of a dose in the second angular position, the drive mechanism 60, the plunger 65 and/or the trigger button 55 may be locked, preventing an additional dose from being administered until the device 5 is returned, temporarily, to the first angular position.

In an exemplary embodiment, the device 5 may include a needle shield (not shown) slidably disposed on the distal part 15 of the housing. The needle shield may have an extended position and a retracted position. Prior to use the needle shield may be in the extended position. When the device 5 is placed on the injection site, the needle shield may move into the retracted position and expose the needle 50. After use the needle shield may lock in the extended position. Preferably, the needle shield is biased in the extended position (e.g., by a spring). If the device 5 is reusable, the needle shield may be unlocked (e.g., by moving the device 5 from the second angular position to the first angular position) prior to delivering a subsequent dose of the medicament.

In an exemplary embodiment, a window (not shown) may be formed on the distal part 15 of the housing to provide visual access to the cartridge 25. Thus, the medicament in the cartridge 25 can be visually examined prior to use.

In use, the device 5 may be oriented in the first angular position, and a cartridge 25 (or syringe) may be inserted into the distal part 15 of the housing. If the device 5 utilizes removable needle assemblies, an unused needle assembly 45 may be coupled to the housing. If the device 5 utilizes a prefilled syringe, a needle sheath may be removed from the needle.

The device 5 may be moved to the second angular position. As the proximal and distal parts 10, 15 of the housing rotate about the hinge 20, the beam 75 returns to its axial orientation, and the operative end 75 displaces the drive mechanism 60 a predetermined distance. A dose may then be set by, for example, rotating the trigger button 55 relative to the proximal part 10 of the housing. Rotation of the trigger button 55 influences the amount of force provided by the drive mechanism 60. When the desired dose is set, the trigger button 55 may be depressed to activate the drive mechanism 60, which applies axial force on the plunger 65 to push the bung 40 and dispense the medicament.

After the dose is delivered, the device 5 may be prevented from applying another dose until the device 5 is returned, temporarily, to the first angular position. In an exemplary embodiment, the beam 70 may lock the plunger 65, the drive mechanism 60 and/or the trigger button 55; returning the device 5 to the first angular position may move the beam 70 to unlock/release the plunger 65, the drive mechanism 60 and/or the trigger button 55. In another exemplary embodiment, the drive mechanism 60 and/or the plunger 65 may need to be reset prior to subsequent use; returning the device 5 to the first angular position may reset the drive mechanism 60 and/or the plunger 65.

In an exemplary embodiment, the device 5 may include a use indicator (not shown). For example, the use indicator may be a window formed on the housing which displays either a red indicia or a green indicia. The red indicia may be displayed after a dose has been delivered and indicate that the device 5 is locked, and a green indicia may be displayed after the device 5 has been returned to the first angular position and is ready for a subsequent injection.

The medicament which may be delivered by the device 5 according to the present invention includes, but is not limited to, a pharmaceutical formulation containing at least one pharmaceutically active compound,
wherein in one exemplary embodiment the pharmaceutically active compound has a molecular weight up to 1500 Da and/or is a peptide, a protein, a polysaccharide, a vaccine, a DNA, a RNA, , an enzyme, an antibody or a fragment thereof, a hormone or an oligonucleotide, or a mixture of the above-mentioned pharmaceutically active compound,
wherein in a further exemplary embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further exemplary embodiment the pharmaceutically active compound comprises at least one peptide for the treatment and/or prophylaxis of diabetes mellitus or complications associated with diabetes mellitus such as diabetic retinopathy,
wherein in a further exemplary embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-N H2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2, des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and p. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand the modifications (additions and/or removals) of various components of the device and/or system and embodiment described herein may be made without departing from the full scope and spirit of the present invention, which encompass such modifications and any and all equivalents thereof.

## Claims

1. A medicament delivery device (5) comprising:
a housing including a proximal part (10) rotatably coupled to a distal part (15);
a drive mechanism (60) disposed with the housing, the drive mechanism (60) adapted to apply force on a plunger (65) for dispensing a medicament; and
a reset mechanism disposed within the housing,
wherein, rotation of the proximal part (10) relative to the distal part (15) from a first angular position to a second angular position activates the reset mechanism to reset the drive mechanism (60).

2. The medicament delivery device (5) according to claim 1, wherein the proximal part (10) is coupled to the distal part (15) via a hinge (20).

3. The medicament delivery device (5) according to any one of the preceding claims,
wherein the proximal part (10) and the distal part (15) are axially aligned in the first angular position and the second angular position.

4. The medicament delivery device (5) according to any one of the preceding claims,
wherein the proximal part (10) is disposed at an angle to the distal part (15) in the first angular position and the proximal part (10) is axially aligned with the distal part (15) in the second angular position.

5. The medicament delivery device (5) according to any one of the preceding claims,
wherein the housing is adapted to receive a cartridge (25) or a syringe.

6. The medicament delivery device (5) according to any one of the preceding claims, further comprising:
a trigger button (55) disposed on the housing, wherein axial movement of the trigger button (55) relative to the housing activates the drive mechanism (60).

7. The medicament delivery device (5) according to claim 6, wherein rotation of the trigger button (55) relative to the housing affects the force applied by the drive mechanism (60).

8. The medicament delivery device (5) according to any one of the preceding claims,
wherein the reset mechanism includes a resilient, elastic beam (70) biasing the proximal part (10) and the distal part (15) to the second angular position.

9. The medicament delivery device (5) according to claim 8, wherein the beam (70) is grounded in the distal part (15) and includes an operative end (75) adapted to engage the drive mechanism (60).

10. The medicament delivery device (5) according to claim 9, wherein the operative end (75) engages the drive mechanism (60) to increase the force applied by the drive mechanism (60) when the proximal part (10) is rotated relative to the distal part (15) from the first angular position to the second angular position.

11. The medicament delivery device (5) according to claims 6 and 9, wherein the beam (70) prevents movement of at least one of the drive mechanism (60), the trigger button (55) and the plunger (65) when the proximal part (10) is rotated relative to the distal part (15) from the second angular position to the first angular position.

12. The medicament delivery device (5) according to any one of the preceding claims, further comprising:
a use indicator displaying a first indicia when the device (5) is ready to be use and a second indicia when the device (5) is locked.

13. The medicament delivery device (5) according to claims 8 and 12, wherein rotation of the proximal part (10) relative to the distal part (15) from the first angular position to the second angular position causes the beam (70) to engage the use indicator to display the first indicia.

14. The medicament delivery device (5) according to claim 1, wherein the medicament is one of insulin, complex carbohydrates, pharmaceutically active peptides, pharmaceutically active carbohydrates, pharmaceutically active nucleic acids, antibodies, a vaccine and a biological material.

15. The medicament delivery device (5) according to anyone of the preceding claims,
wherein the drive mechanism (45) is one or more of a spring, a pneumatic device a hydraulic device, a magnetic drive and a motor.
